# EUROPEAN PATENT SPECIFICATION

(11) **EP 0 423 221 B1**
(45) Date of publication and mention of the grant of the patent: **25.09.1996**
(21) Application number: 89908485.9
(22) Date of filing: 06.07.1989
(51) Int. Cl.: C07D 233/92, C07D 401/12, C07D 403/12, C07D 413/12, C07D 417/12, C07D 471/04, C07D 473/38, C07D 513/04, A61K 31/33, A61K 31/41

(54) **IMMUNOSUPPRESSIVE AGENTS**
IMMUNSUPPRESSIVES AGENS
AGENTS IMMUNOSUPPRESSEURS

(30) Priority: 06.07.1988 GB 8816123
(43) Date of publication of application: 24.04.1991
(73) Proprietor: THE UNIVERSITY OF SHEFFIELD, Sheffield, South Yorkshire S10 2TN (GB)
(72) Inventor: JONES, Neville, Sheffield S11 7LB (GB); MADDOCKS, John, L., Sheffield S11 9BQ (GB)
(74) Representative: Fyles, Julie Marie
(86) International application number: GB8900771
(87) International publication number: WO9000549

(56) References cited:
- EP-A- 0 260 057
- EP-A- 0 293 742
- GB-A- 878 646
- US-A- 3 862 061
- CHEMICAL ABSTRACTS, vol. 105, no. 3, July 21, 1986, Columbus, Ohio, US; VAN CANEGHEM P.et al.: "Radiosensitizing properties of an azothioprine analog",page 343, abstract no. 21069f
- CHEMICAL ABSTRACTS, vol. 96, no. 13, March 29, 1982, Columbus, Ohio, US;MROCZKIEWICZ A.: "Synthesis of sulfides derived from nitroimidazole", page 700,abstract-no. 104146a
- CHEMICAL ABSTRACTS, vol. 107, no. 23, December 7, 1987, Columbus, Ohio, US;BALONIAK S. et al.: "Preparation of 6-[(1,2-dimethyl-4-nitro-5-imidazolyl)thio]purine", page 564, abstract-no. 217370h
- CHEMICAL ABSTRACTS, vol. 85, no. 1, July 5, 1976, Columbus, Ohio, US; MOTONOBUI. et al.: "4-Mercaptopyrazolo[3,4-d]pyrimidine derivative", page 459,abstract-no. 5680m

## Description

This invention relates to immunosuppressive agents.

The purine derivative azathioprine interfers with cell replication, and has immunosuppressive and antileukamic properties. It is used extensively as a drug to treat a wide range of chronic inflammatory diseases in which immune mechanisms are involved such as rheumatoid arthritis; chronic active hepatitis; kidney disease; skin disease; and multiple sclerosis. It is also used to inhibit the rejection of transplanted organs such as kidneys. However, its beneficial therapeutic effects are compromised by the fact that it is toxic to bone marrow. It is the object of the present invention to provide an immunosuppressive agent which does not have this disadvantage.

According to one aspect of the invention there is provided a compound for use as an immunosuppressive agent, said compound having the formula: 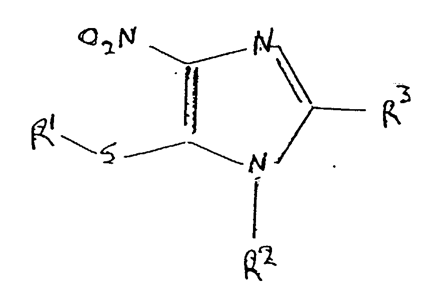 in which
R¹ comprises a heterocyclic ring system, a homocyclic ring system, a combined heterocyclic and homocyclic ring system, alkoxycarbonylalkyl or aryloxycarbonylalkyl group and wherein
R¹ does not comprise 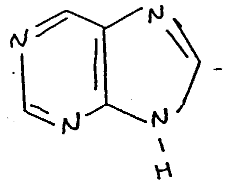 or isomers thereof.
R² is alkyl, aryl or hydrogen and
R³ is hydrogen, alkyl, aryl or nitroaryl

Preferably
R² is hydrogen, methyl, ethyl or propyl; and
R³ is hydrogen, methyl or 4-nitrophenyl.

According to a preferred embodiment of the invention said compounds which are used as immunosuppressive agents have the following formula: 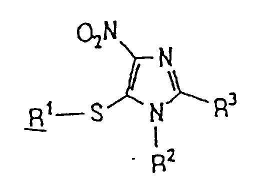 in which
R² is hydrogen, or methyl or ethyl or propyl; and
R³ is hydrogen or methyl or 4-nitrophenyl,
   and in which R¹ may be one of the following: 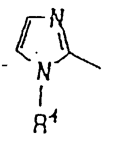 in which
R⁴ is hydrogen or methyl or ethyl or propyl or phenyl or 1-naphthyl 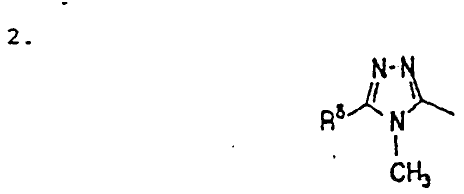 in which
R⁵ is hydrogen or trifluoromethyl or phenyl or 4-chlorophenyl or 4-methoxyphenyl or 3-pyridyl 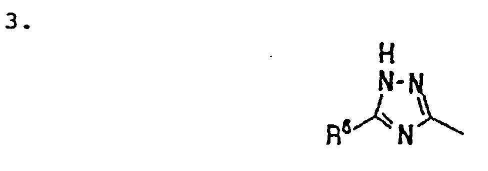 in which
R⁶ is hydrogen or phenyl or chlorophenyl or 4-methoxyphenyl 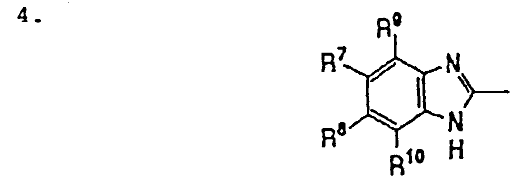 in which
R¹⁰ is hydrogen or an alkoxy group; and
R⁷ is hydrogen, alkyl, nitro, halide or an alkoxy group; and
R⁸ is hydrogen, halide or an alkoxy group; and
R⁹ is hydrogen or an alkoxy group 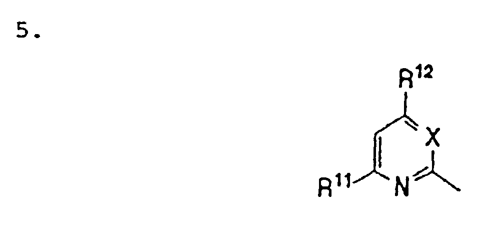 in which
R¹¹ is hydrogen, methyl, or hydroxyl; and
R¹² is hydrogen or methyl; and
X is nitrogen or C-H 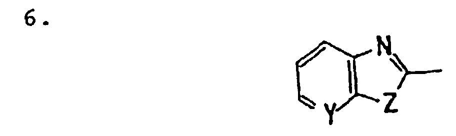 in which
Z is oxygen or sulphur or N-H; and
Y is C-H or nitrogen 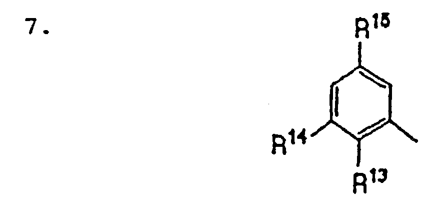 in which
R¹³ is hydrogen or an amino or carboxyl group; and
R¹⁴ is hydrogen or methyl; and
R¹⁵ is hydrogen or methyl 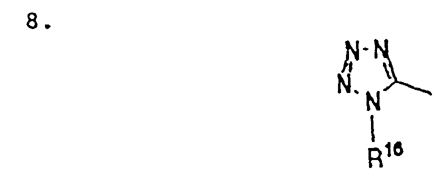 in which
R¹⁶ is alkyl or aryl 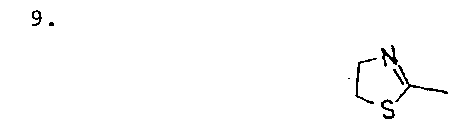 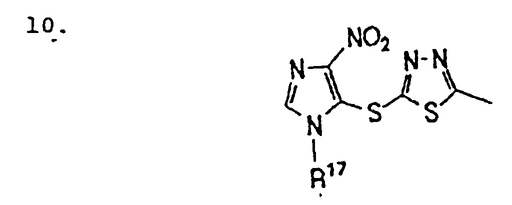 in which
R¹⁷ is alkyl or aryl 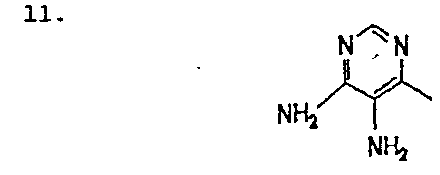 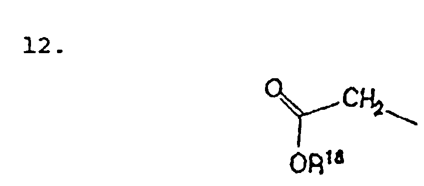 in which R¹⁸ is alkyl or aryl

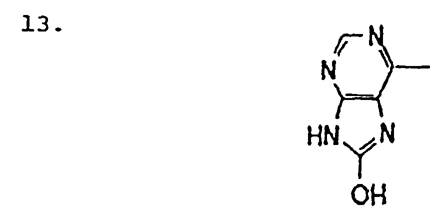

Specific illustrative examples of the first embodiment of the invention are as follows:

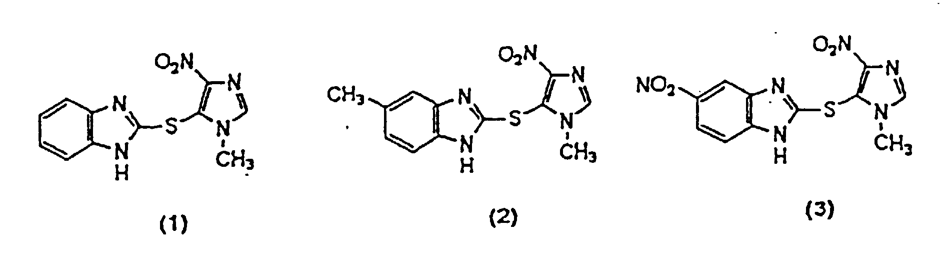

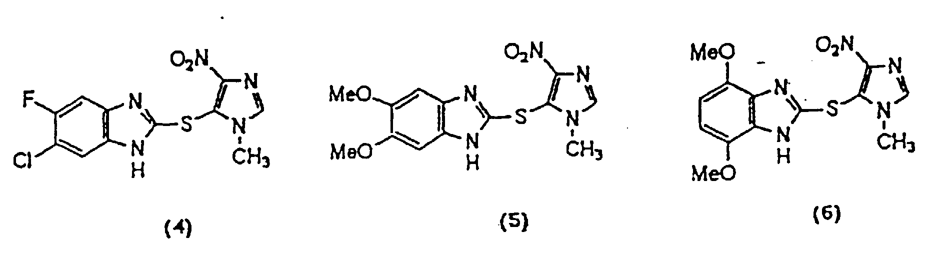

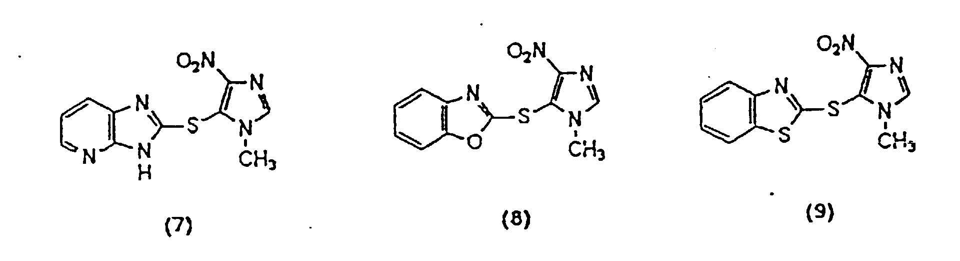

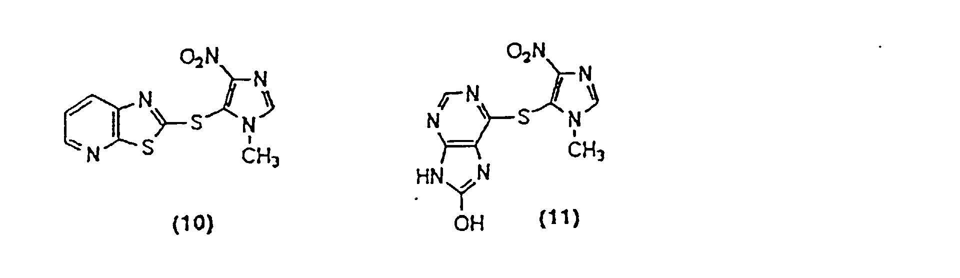

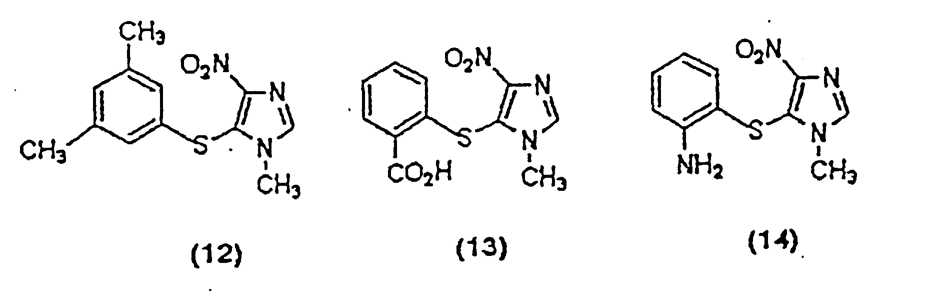

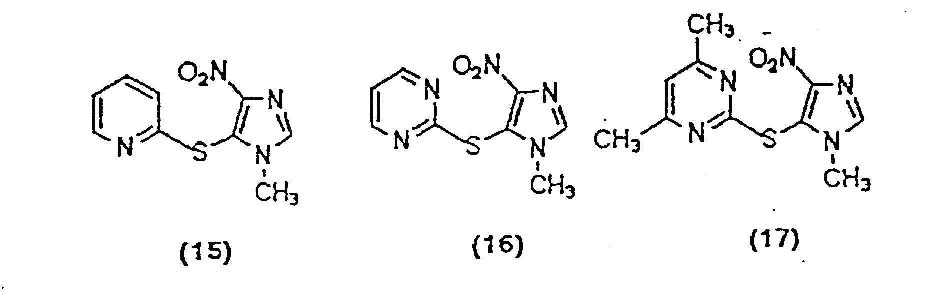

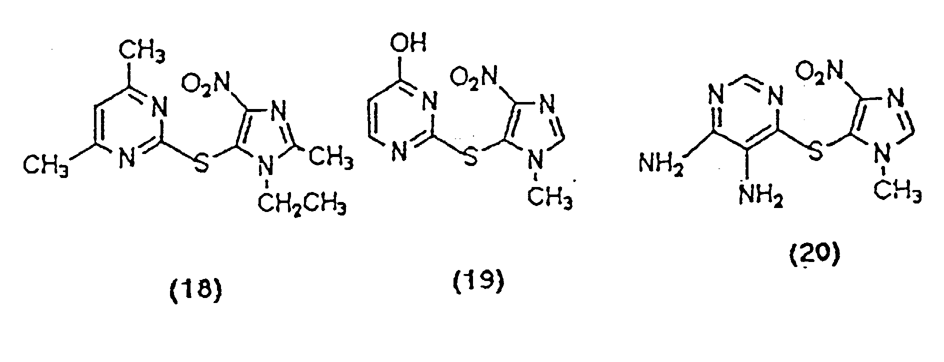

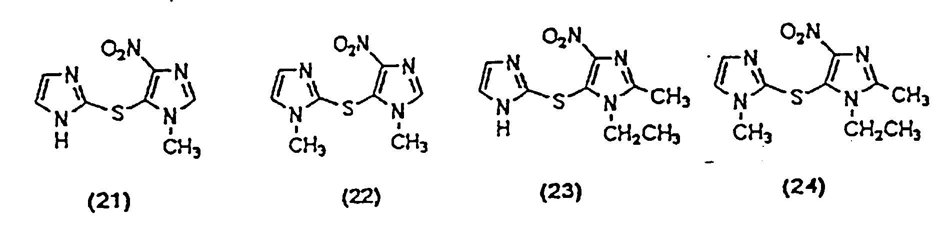

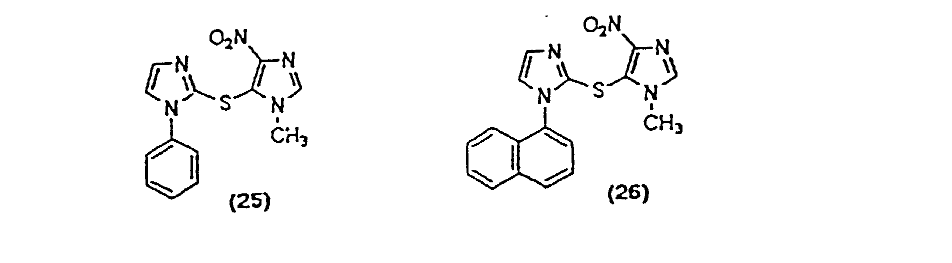

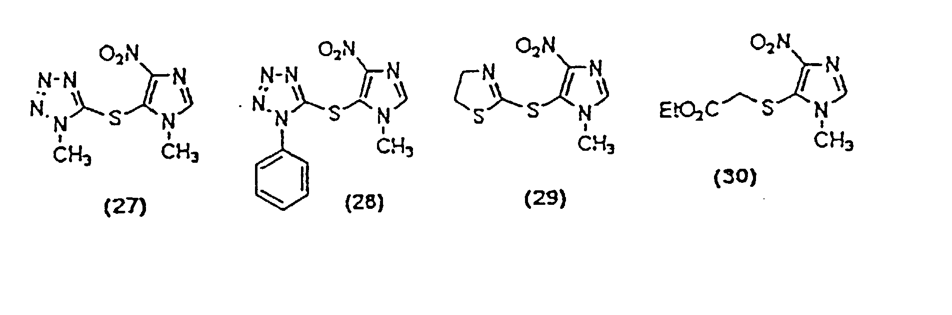

Some of the compounds, as referred to above are previously unknown.

Therefore, according to a further aspect of the invention there are provided compounds comprising the following formula: 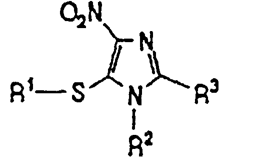 wherein the molecular groups R¹, R², and R³ are defined as one of the following:
1.
   R¹ is defined as the following: 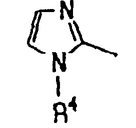 in which
   R⁴ is hydrogen or methyl;
   R² is ethyl; and
   R³ is methyl
      OR
   R² is methyl and R³ is hydrogen; and
   R⁴ is phenyl or 1-naphthyl
      OR
2.
   R¹ is defined as the following: 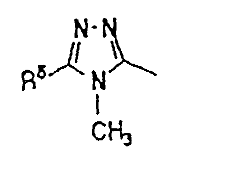 in which
   R²,R³, and R⁵ are one of the following combinations:
   R² is methyl, R³ is 4-nitrophenyl, and R⁵ is hydrogen; or
   R² is methyl, R³ is hydrogel, and R⁵ is trifluoromethyl; or
   R² is ethyl, R³ is methyl, and R⁵ is hydrogen; or
   R² is methyl, R³ is hydrogen, and R⁵ is phenyl; or
   R² is methyl, R³ is hydrogen, and R⁵ is 4-chlorophenyl; or
   R² is methyl, R³ is hydrogen, and R⁵ is 4-methoxyphenyl; or
   R² is methyl, R³ is hydrogen, and R⁵ is 3-pyridyl
      OR
3.
   R¹ is defined as the following: 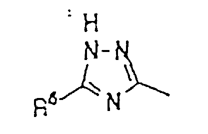 in which
   R² is methyl; and
   R³ is hydrogen; and
   R⁶ is hydrogen or phenyl or 4-chlorophenyl or 4-methoxyphenyl
      OR
4.
   R¹ is defined as the following: 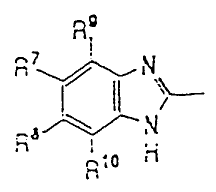 in which
   R² is methyl; and
   R³ is hydrogen, and
   R⁷, R⁸, R⁹, R¹⁰ are one of the following combinations:
   R⁸, R⁹, R¹⁰ are all hydrogen and R⁷ is methyl; or
   R⁸, R⁹, R¹⁰ are all hydrogen and R⁷ is a nitro group; or
   R⁹ and R¹⁰ are both hydrogen, R⁷ is fluorine, and R⁸ is chlorine; or
   R⁹ and R¹⁰ are both hydrogen, and R⁷ and R⁸ are both methoxy groups; or
   R⁷ and R⁸ are both hydrogen and R⁹ and R¹⁰ are both methoxy groups
      OR
5.
   R¹ is defined as the following: 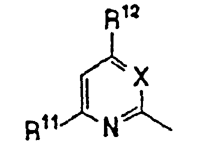 in which
   R² is methyl; and
   R³ is hydrogen; and
   R¹¹,R¹² and X are defined as one of the following combinations:
   R¹¹ and R¹² are both hydrogen, and X is C-H; or
   R¹¹ is a hydroxy group, R¹² is hydrogen, and X is nitrogen
      OR
   R² is ethyl; and
   R³, R¹¹ and R¹² are all methyl; and
   X is nitrogen
      OR
6.
   R¹ is defined as the following: 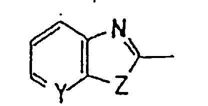 in which
   R² is methyl; and
   R³ is hydrogen; and
   Y and Z are defined as one of the following combinations:
   Y is C-H and Z is oxygen; or
   Y is nitrogen and Z is sulphur; or
   Y is nitrogen and Z is N-H
      OR
7.
   R¹ is defined as the following: 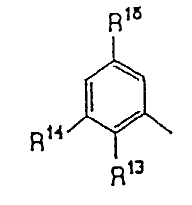 in which
   R² is methyl; and
   R³ is hydrogen; and
   R¹³,R¹⁴, and R¹⁵ are defined in either of the following combinations:
   R¹³ is hydrogen, and R¹⁴ and R¹⁵ are both methyl; or
   R¹³ is carboxyl, and R¹⁴ and R¹⁵ are both hydrogen
      OR
8.
   R¹ is defined as the following: 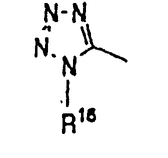 in which
   R² is methyl; and
   R³ is hydrogen; and
   R¹⁶ is methyl or phenyl
      OR
9. R¹ is defined as the following: 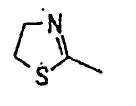 OR
10. R¹ is defined as the following: 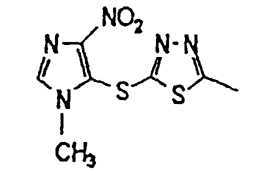 OR
11. R¹ is defined as the following;

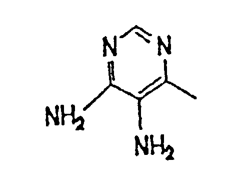

Referring now to Reaction Scheme 1, azathioprine I was originally designed as a "pro-drug" for 6-mercaptopurine II, to which it is rapidly converted by red blood cells. 6-mercaptopurine II has immunosuppressive properties, but the mechanism of its action is unclear.

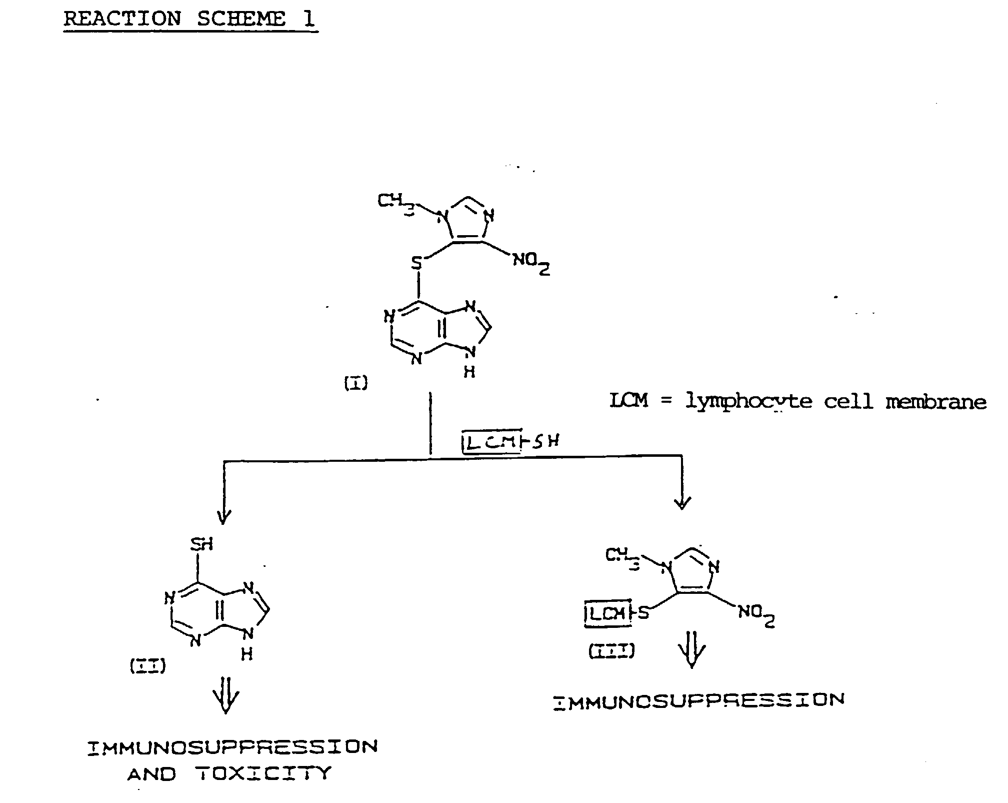

It would appear that the immunosuppressive action of azathioprine I is due not solely to prior conversion of 6-mercaptopurine II in the body, but to the immunosuppressive action of azathioprine I itself. The mechanism of action of azathioprine I differs from that of 6-mercaptopurine II. There is strong evidence to suggest that 6-mercaptopurine II is converted in vivo into a nucleotide metabolite that is associated with bone marrow toxicity. There is no evidence to suggest that metabolites derived from the 1-methyl-4-nitroimidazole moiety of azathioprine give rise to toxicity.

It is thought that azathioprine I alkylates thiol groups in the lymphocyte cell membrane, most probably by a process of addition-elimination with the consequent release of 6-mercaptopurine II. It is further supposed that the alkylation of the lymphocyte results in immunosuppression, whilst the 6-mercapcopurine II gives rise, as stated above, via its nucleotide metabolite, to bone marrow toxicity. Support for this hypothesis is provided by the fact that lymphocyte cell membranes are richly endowed with thiol groups, and by the known propensity of azathioprine to react with thiols both in vitro and in vivo.

5-(1-alkyl-4-nitroimidazole) derivatives VI of relatively non toxic alkyl thiols or aryl thiols V are therefore suitable candidates for evaluation as good, relatively non toxic immunosuppressive agents, as is shown in Reaction Scheme 2.

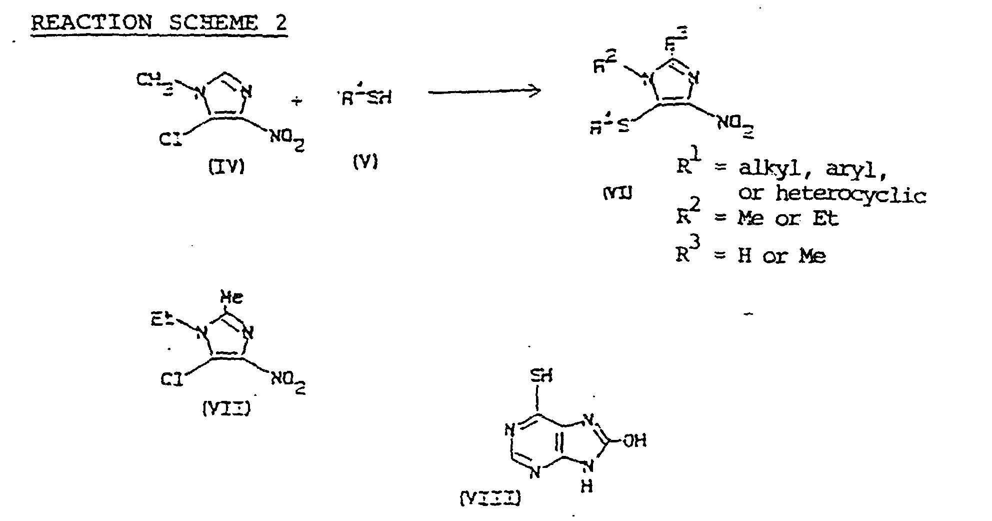

Examples of immunosuppressive agents of this kind are shown previously in examples 1-30. It is not intended that the invention be limited to these illustrative examples.

Compounds 1 to 17, 19 to 22, and 25 to 30, may be synthesised, for example, by reaction of 5-chloro-1-methyl-4-nitroimidazole IV with the appropriate thiol V and potassium carbonate in a suitable solvent such as, for example, tetrahydrofuran, dimethylformamide, or water. Compounds 18, 23, and 24 may be synthesised, for example from 5-chloro-1-ethyl-2-methyl-4-nitroimidazole Vll and the appropriate thiol under suitable conditions, such as, for example, in acetone in the presence of potassium carbonate.

Fifteen of these compounds were screened for immunosuppressive activity by means of the human mixed lymphocyte reaction, which is well known and need not be further described here, the results of which are shown in Table 1.

**TABLE 1**

| Compound | Concentration of Solution (µM) | % Inhibition of ³M-Thymidine |
|---|---|---|
| I (azathioprine) | 25 | 79 |
| 21 | 10 | 89 |
| 22 | 10 | 82 |
| 1 | 36 | 98 |
| 2 | 25 | 86 |
| | | |
| 4 | 50 | 59 |
| 5 | 25 | 94 |
| 6 | 25 | 41 |
| 15 | 25 | 76 |
| 16 | 25 | 47 |
| 19 | 25 | 29 |
| 18 | 25 | 47 |
| 8 | 25 | 87 |
| 9 | 25 | 27 |
| 13 | 25 | 34 |
| 30 | 25 | 65 |

The individual figures in the right hand column of the above table are directly proportioned to immunosuppressive activity.

As can be seen from Table 1 all of the compounds screened for immunosuppressive activity displayed significant immunosuppression, and five were substantially more active than azathioprine at the same concentration (25µM).

Table 2 shows the measure of the immunosuppressive activity and toxicity of compounds 21,22 and 11.

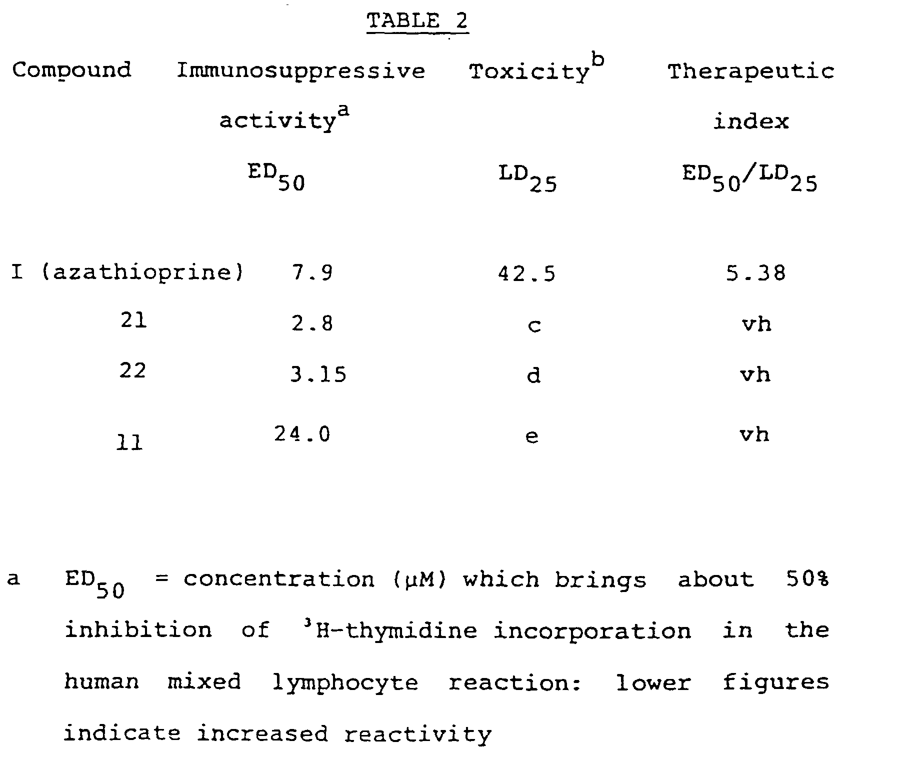

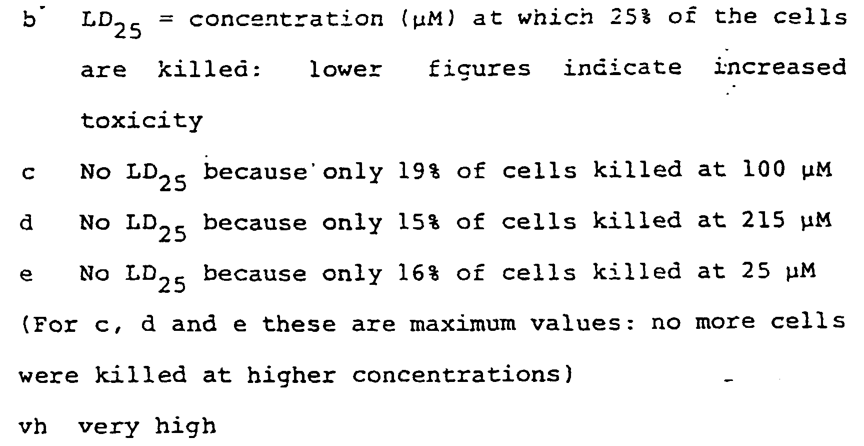

Referring now to Table 2, imidazole derivatives 21 and 22 are shown to be substantially more immunosuppressive than azathioprine and also considerably less toxic to lymphocytes. Therapeutic indices for these compounds are therefore at least an order of magnitude greater than for azathioprine.

The compound 11 in which the 6-mercaptopurine moiety of azathioprine is replaced by 8-hydroxy-6-mercaptopurine moiety, is less immunosuppressive than azathioprine according to the mixed human lymphocyte reaction, as is shown in Table 2, but is much less toxic to the lymphocytes. The therapeutic index of compound 11 is therefore much greater than that of azathioprine, according to these tests. The significance of this result resides in the fact that 8-hydroxy-6-mercaptopurine VIII is known to be a non toxic metabolite of azathioprine, and its metabolic fate is known.

It is to be understood that the above described examples are for illustration only.

## Claims

1. A compound for use as an immunosuppressive agent, said compound having the formula: 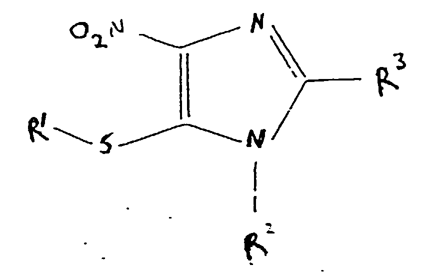 in which
R¹ comprises a heterocyclic ring system, a homocyclic ring system a combined heterocyclic and homocyclic ring system or alkoxycarbonylalkyl group or aryloxycarbonyalkyl group and wherein R¹ does not comprise 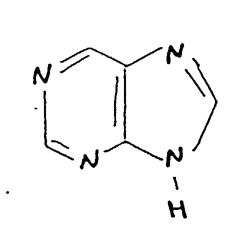 or isomers thereof
R₂ is hydrogen, alkyl or aryl; and
R3 is hydrogen alkyl, aryl and nitroaryl.

2. A compound for use as an immunosuppressive agent as claimed in claim 1 in which R² is hydrogen, methyl, ethyl or propyl; and R³ is hydrogen, methyl or 4-nitrophenyl.

3. A compound for use as an immunosuppressive agent as claimed in claims 1 or 2 having the following formula: 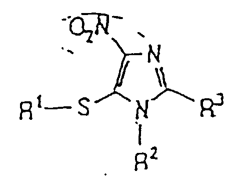 in which
R² is hydrogen, or methyl or ethyl or propyl, and
R³ is hydrogen or methyl or 4-nitrophenyl,
and in which R¹ may be one of the following 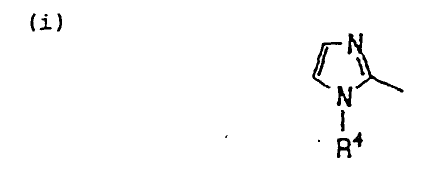 in which
R⁴ is hydrogen or methyl or ethyl or propyl or phenyl or 1-naphthyl
OR 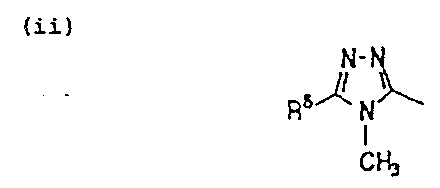 in which
R⁵ is hydrogen or trifluoromethyl or phenyl or 4-chlorophenyl or 4-methoxyphenyl or 3-pyridyl
OR 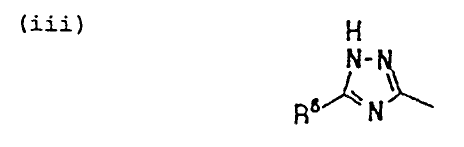 in which
R⁶ is hydrogen or phenyl or chlorophenyl or 4-methoxyphenyl
OR 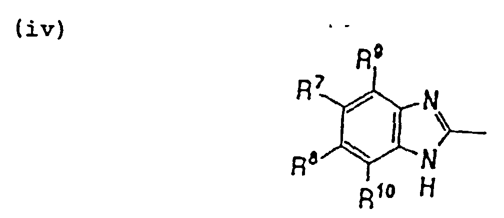 in which
R¹⁰ is hydrogen or an alkoxy group; and
R⁷ is hydrogen, alkyl, nitro, halide or an alkoxy group; and
R⁸ is hydrogen, halide or an alkoxy group; and
R⁹ is hydrogen or an alkoxy group
OR 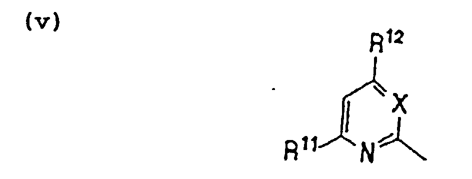 in which
R¹¹ is hydrogen, methyl, or hydroxyl; and
R¹² is hydrogen or methyl; and
X is nitrogen or C-H
OR 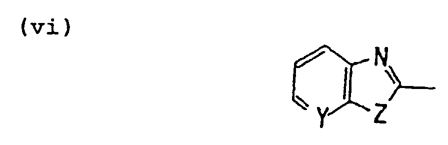 in which
Z is oxygen or sulphur or N-H; and
Y is C-H or nitrogen
OR 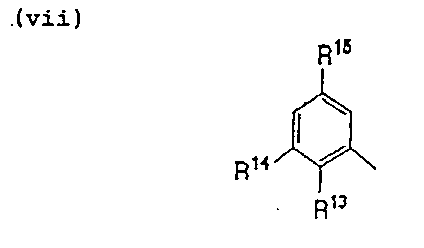 in which
R¹³ is hydrogen or an amino or carboxyl group; and
R¹⁴ is hydrogen or methyl; and
R¹⁵ is hydrogen or methyl
OR 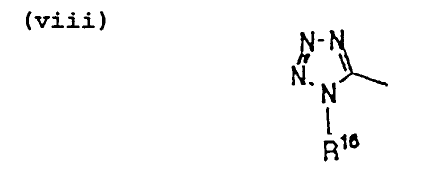 in which
R¹⁶ is alkyl or aryl
OR 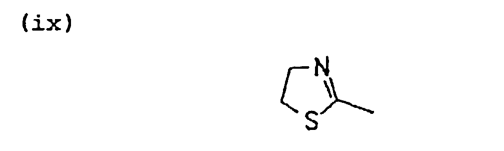 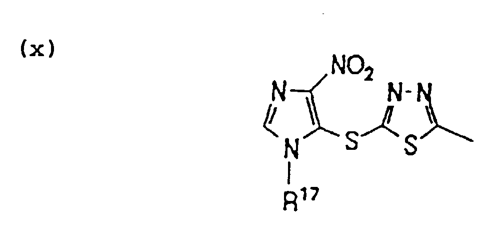 in which
R¹⁷ is alkyl or aryl
OR 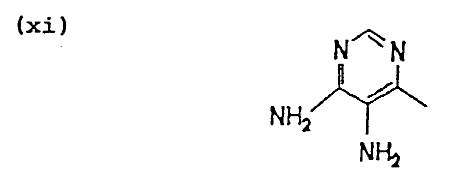 OR 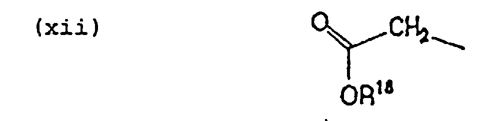 in which R¹⁸ is alkyl or aryl
OR

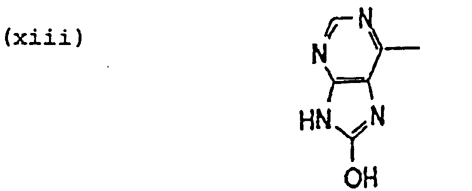

4. A compound comprising the following formula: 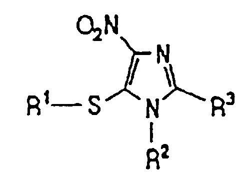 wherein the molecular groups R¹, R² and R³ are defined as one of the following:
(i) R¹ is defined as the following: 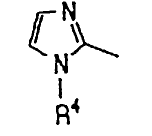 in which
R⁴ is hydrogen or methyl;
R² is ethyl; and
R³ is methyl
OR
R² is methyl and R³ is hydrogen; and
R⁴ is phenyl or 1-naphthyl
OR
(ii) R¹ is defined as the following: 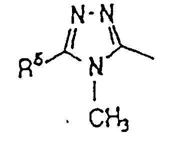 in which
R²,R³ and R⁵ are one of the following combinations:
R² is methyl, R³ is 4-nitrophenyl, and R⁵ is hydrogen, or
R² is methyl, R³ is hydrogen, and R⁵ is trifluoromethyl;or
R² is ethyl, R³ is methyl, and R⁵ is hydrogen; or
R² is methyl, R³ is hydrogen, and R⁵ is phenyl; or
R² is methyl, R³ is hydrogen, and R⁵ is 4-chlorophenyl; or
R² is methyl, R³ is hydrogen, and R⁵ is 4-methoxyphenyl;or
R² is methyl, R³ is hydrogen, and R⁵ is 3-pyridyl
OR
(iii) R¹ is defined as the followings 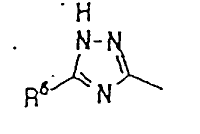 in which
R² is methyl; and
R³ is hydrogen; and
R⁶ is phenyl or 4-chlorophenyl or
4-methoxyphenyl
OR
(iv) R¹ is defined as the following: 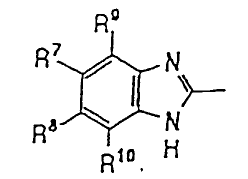 in which
R² is methyl; and
R³ is hydrogen; and
R⁷, R⁸, R⁹, R¹⁰ are one of the following combinations;
R⁹ and R¹⁰ are both hydrogen, and R⁷ and R⁸ are both methoxy groups; or
R⁸, R⁹, R¹⁰ are all hydrogen and R⁷ is methyl; or
R⁸, R⁹, R¹⁰ are all hydrogen and R⁷ is a nitro group; or
R⁹ and R¹⁰ are both hydrogen, R⁷ is fluorine, and R⁸ is chlorine; or
R⁷ and R⁸ are both hydrogen and R⁹ and R¹⁰ are both methoxy groups
OR
(v) R¹ is defined as the following: 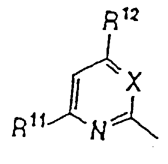 in which
R² is methyl; and
R³ is hydrogen; and
R¹¹ and R¹² and X are defined as one of the following combinations:
R¹¹ is a hydroxy group, R¹² is hydrogen, and X is nitrogen
OR
R² is ethyl; and
R³,R¹¹ and R¹² are all methyl; and
X is nitrogen
OR
(vi) R¹ is defined as the following: 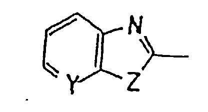 in which
R² is methyl; and
R³ is hydrogen; and
Y and Z are defined as one of the following combinations;
Y is C-H and Z is oxygen; or
Y is nitrogen and Z is sulphur; or
Y is nitrogen and Z is N-H
OR
(vii) R¹ is defined as the following: 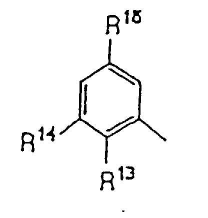 in which
R² is methyl; and
R³ is hydrogen; and
R¹³'R¹⁴ and R¹⁵ are defined in either of the following combinations,
R¹³ is hydrogen, and R¹⁴ and R¹⁵ are both methyl;
OR
(viii) R¹ is defined as the following: 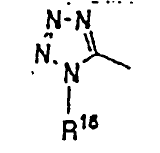 in which
R² is methyl; and
R³ is hydrogen; and
R¹⁶ is methyl or phenyl
OR
(ix) R¹ is defined as the following: 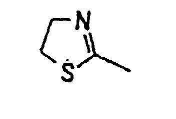 OR
(x) R¹ is defined as the following: 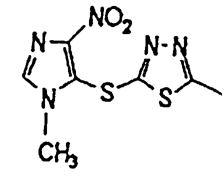 OR
(xi) R¹ is defined as the following:

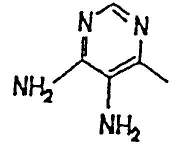

5. A method of producing a compound as claimed in any preceding claim by the reaction of 5-chloro-4-nitroimidazole or derivatives thereof with a thiol in the presence of solvent and carbonate.

## Patentansprüche

1. Eine Verbindung zur Verwendung als immunosuppressives Agens mit folgender Formel: 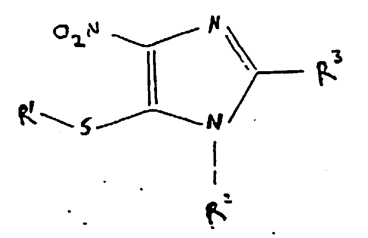 wobei
R¹ aus einem heterocyclischen Ringsystem, einem homocyclischen Ringsystem, einer Kombination aus heterocyclischem und homocyclischem Ringsystem oder einer Alkoxycarbonylalkylgruppe oder einer Aryloxycarbonylalkylgruppe besteht und R¹ nicht 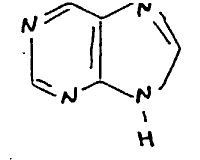 bzw. Isomere davon enthält,
R² Wasserstoff, Alkyl oder Aryl ist und
R³ Wasserstoff, Alkyl, Aryl und Nitroaryl ist.

2. Eine Verbindung zur Verwendung als immunosuppressives Agens wie in Anspruch 1, wobei R² Wasserstoff, Methyl, Ethyl oder Propyl und R³ Wasserstoff, Methyl oder 4-Nitrophenyl ist.

3. Eine Verbindung zur Verwendung als immunosuppressives Agens wie in Anspruch 1 oder 2 mit folgender Formel: 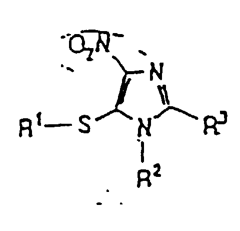 wobei
R² Wasserstoff oder Methyl oder Ethyl oder Propyl ist und
R³ Wasserstoff oder Methyl oder 4-Nitrophenyl ist,
und R¹ bestehen kann aus: 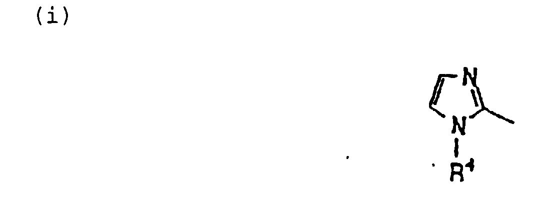 wobei
R⁴ Wasserstoff oder Methyl oder Ethyl oder Propyl oder Phenyl oder 1-Naphthyl ist
ODER 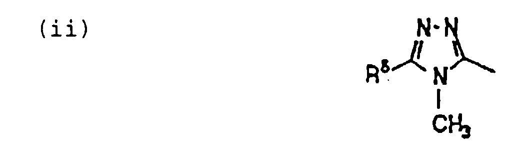 wobei
R⁵ Wasserstoff oder Trifluormethyl oder Phenyl oder 4-Chlorphenyl oder 4-Methoxyphenyl oder 3-Pyridyl ist
ODER 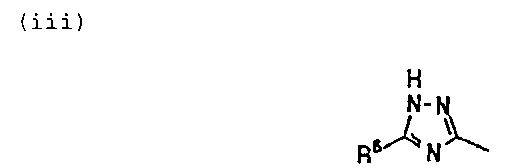 wobei
R⁶ Wasserstoff oder Phenyl oder Chlorphenyl oder 4-Methoxyphenyl ist
ODER 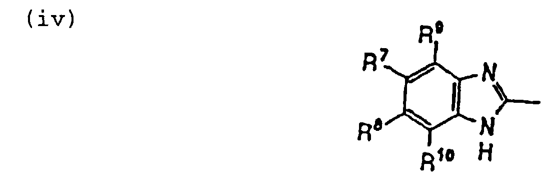 wobei
R¹⁰ Wasserstoff oder eine Alkoxylgruppe ist und
R⁷ Wasserstoff, Alkyl, Nitro, Halogenid oder eine Alkoxylgruppe ist und
R⁸ Wasserstoff, Halogenid oder eine Alkoxylgruppe ist und
R⁹ Wasserstoff oder eine Alkoxylgruppe ist
ODER 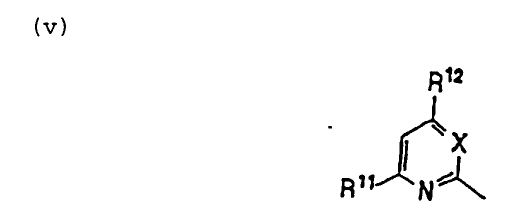 wobei
R¹¹ Wasserstoff, Methyl oder Hydroxyl ist und
R¹² Wasserstoff oder Methyl ist und
X Stickstoff oder C-H ist
ODER 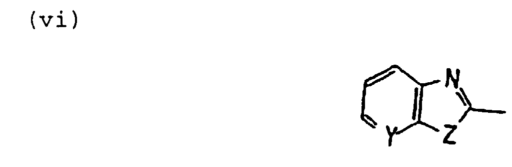 wobei
Z Sauerstoff oder Schwefel oder N-H ist und
Y C-H oder Stickstoff ist
ODER 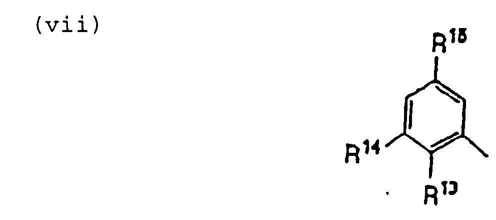 wobei
R¹³ Wasserstoff oder eine Amino- oder Carboxylgruppe ist und
R¹⁴ Wasserstoff oder Methyl ist und
R¹⁵ Wasserstoff oder Methyl ist
ODER 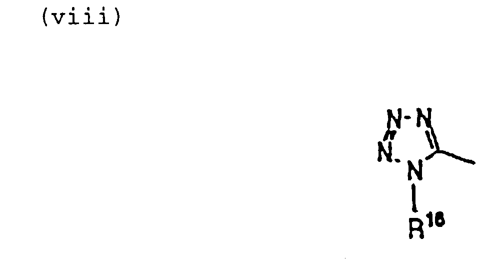 wobei
R¹⁶ Alkyl oder Aryl ist
ODER 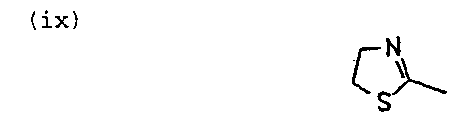 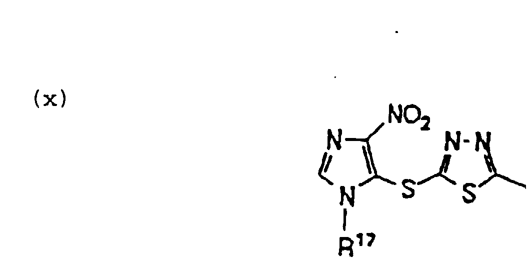 wobei
R¹⁷ Alkyl oder Aryl ist
ODER 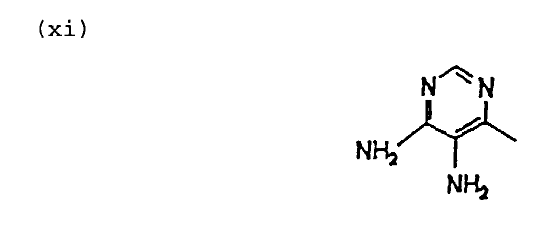 ODER 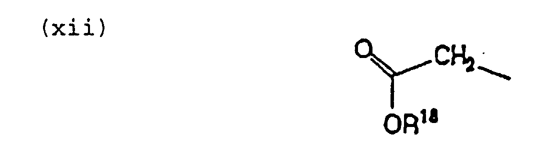 wobei R¹⁸ Alkyl oder Aryl ist
ODER

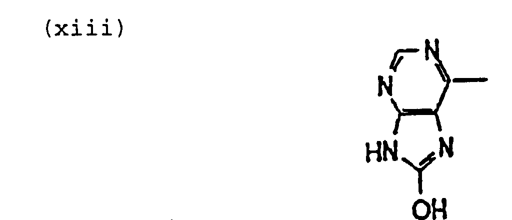

4. Eine Verbindung mit der folgenden Formel: 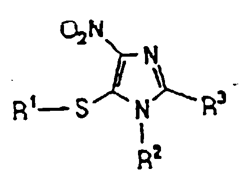 wobei die Molekülgruppen R¹, R² und R³ wie folgt definiert sind:
(i) R¹ ist definiert als: 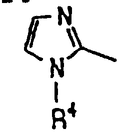 wobei
R⁴ Wasserstoff oder Methyl ist
R² Ethyl ist und
R³ Methyl ist
ODER
R² Methyl und R³ Wasserstoff ist und
R⁴ Phenyl oder 1-Naphthyl ist
ODER
(ii) R¹ ist definiert als: 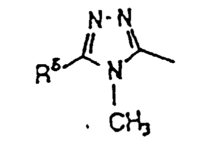 wobei
R², R³ und R⁵ wie folgt kombiniert werden können:
R² ist Methyl, R³ ist 4-Nitrophenyl und R⁵ ist Wasserstoff, oder
R² ist Methyl, R³ ist Wasserstoff und R⁵ ist Trifluormethyl, oder
R² ist Ethyl, R³ ist Methyl und R⁵ ist Wasserstoff, oder
R² ist Methyl, R³ ist Wasserstoff und R⁵ ist Phenyl, oder
R² ist Methyl, R³ ist Wasserstoff und R⁵ ist 4-Chlorphenyl, oder
R² ist Methyl, R³ ist Wasserstoff und R⁵ ist 4-Methoxyphenyl, oder
R² ist Methyl, R³ ist Wasserstoff und R⁵ ist 3-Pyridyl
ODER
(iii) R¹ ist definiert als: 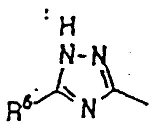 wobei
R² Methyl ist und
R³ Wasserstoff ist und
R⁶ Phenyl oder 4-Chlorphenyl oder 4-Methoxyphenyl ist
ODER
(iv) R¹ ist definiert als: 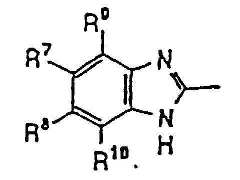 wobei
R² Methyl ist und
R³ Wasserstoff ist und
R⁷, R⁸, R⁹ und R¹⁰ wie folgt kombiniert werden können:
R⁹ und R¹⁰ sind beide Wasserstoff, und R⁷ und R⁸ sind beide Methoxylgruppen, oder
R⁸, R⁹, R¹⁰ sind alle Wasserstoff, und R⁷ ist Methyl, oder
R⁸, R⁹, R¹⁰ sind alle Wasserstoff, und R⁷ ist eine Nitrogruppe, oder
R⁹ und R¹⁰ sind beide Wasserstoff, R⁷ ist Fluor, und R⁸ ist Chlor, oder
R⁷ und R⁸ sind beide Wasserstoff, und R⁹ und R¹⁰ sind beide Methoxylgruppen
ODER
(v) R¹ ist definiert als: 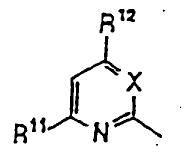 wobei
R² Methyl ist und
R³ Wasserstoff ist und
R¹¹ und R¹² und X wie folgt kombiniert werden können:
R¹¹ ist eine Hydroxylgruppe, R¹² ist Wasserstoff, und X ist Stickstoff
ODER
R² Ethyl ist und
R³, R¹¹ und R¹² Methyl sind und
X Stickstoff ist
ODER
(vi) R1 ist definiert als: 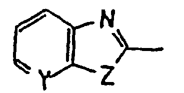 wobei
R² Methyl ist und
R³ Wasserstoff ist und
Y und Z wie folgt kombiniert werden können:
Y ist C-H, und Z ist Sauerstoff, oder
Y ist Stickstoff, und Z ist Schwefel, oder
Y ist Stickstoff, und Z ist N-H
ODER
(vii) R¹ ist definiert als: 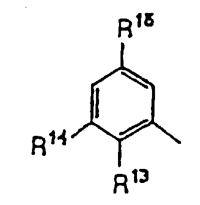 wobei
R² Methyl ist und
R³ Wasserstoff ist und
R¹³, R¹⁴ und R¹⁵ wie folgt kombiniert werden können:
R¹³ ist Wasserstoff, und R¹⁴ sowie R¹⁵ sind beide Methyl,
ODER
(viii) R¹ ist definiert als: 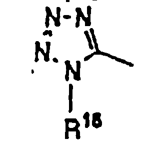 wobei
R² Methyl ist und
R³ Wasserstoff ist und
R¹⁶ Methyl oder Phenyl ist
ODER
(ix) R¹ ist definiert als: 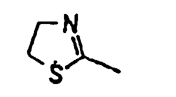 ODER
(x) R¹ ist definiert als: 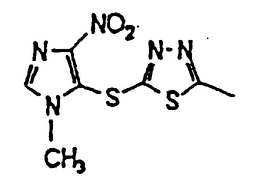 ODER
(xi) R¹ ist definiert als:

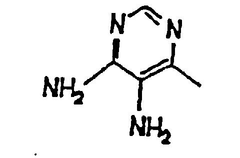

5. Ein Verfahren zur Herstellung einer Verbindung, wie sie in einem der oben genannten Ansprüche beschrieben wird, durch Reaktion von 5-Chlor-4-nitroimidazol oder von Derivaten davon mit einem Thiol in Anwesenheit eines Solvens und Carbonat.

## Revendications

1. Un composé pouvant être utilisé comme agent immunosuppresseur, ledit composé ayant la formule suivante : 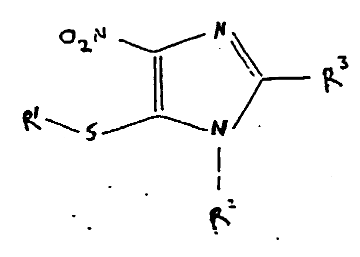 dans laquelle
R¹ contient une chaîne hétérocyclique, une chaîne homocyclique, une chaîne combinée hétérocyclique et homocyclique ou un groupe alkoxycarbonyalcoyle ou un groupe aryloxycarbonylalcoyle et où R¹ ne contient pas 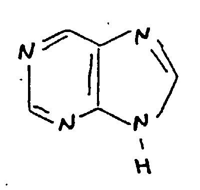 ou ses isomères
R² est de l'hydrogène, un alcoyle ou un aryle ; et
R³ est de l'hydrogène alcoyle, aryle et nitroaryle.

2. Un composé pouvant être utilisé comme agent immunosuppresseur comme revendiqué dans la revendication 1 et dans lequel R² est de l'hydrogène, du méthyle, de l'éthyle ou du propyle ; et R³ est de l'hydrogène, du méthyle ou du 4-nitrophényle.

3. Un composé pouvant être utilisé comme agent immunosuppresseur comme revendiqué dans les revendications 1 et 2 et ayant la formule suivante : 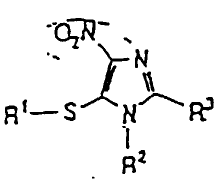 dans laquelle
R² est de l'hydrogène, du méthyle, de l'éthyle ou du propyle et R³ est de l'hydrogène ou du méthyle ou du 4-nitrophényle,
et dans laquelle R¹ peut être l'un des éléments suivants : 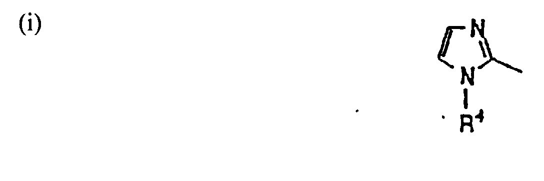 dans lequel
R⁴ est de l'hydrogène, du méthyle, de l'éthyle, du propyle, du phényle ou du 1-naphtyle
OU 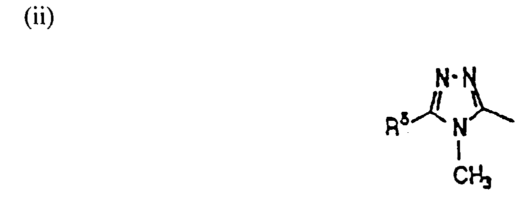 dans lequel
R⁵ est de l'hydrogène, du trifluorométhyle, du phényle, du 4-chlorophényle, du 4-méthoxyphényleou du 3-pyridyle
OU 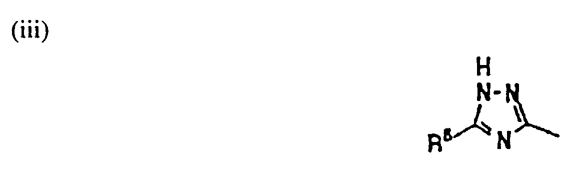 dans lequel
R⁶ est de l'hydrogène, du phényle, du chlorophényle ou du 4-méthoxyphényle
OU 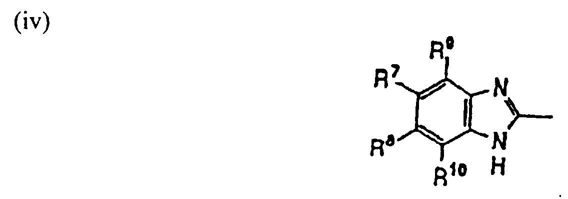 dans lequel
R¹⁰ est de l'hydrogène ou un groupe alkoxy ; et
R⁷ est de l'hydrogène, un alcoyle, un groupe nitré, un halogène ou un groupe alkoxy ; et
R⁸ est de l'hydrogène, un halogène ou un groupe alkoxy ; et
R⁹ est de l'hydrogène ou un groupe alkoxy
OU 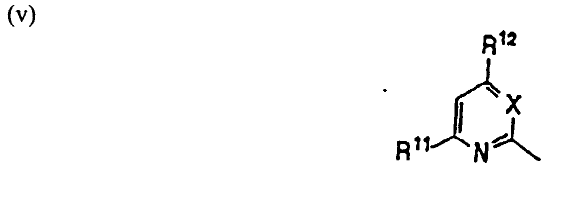 dans lequel
R¹¹ est de l'hydrogène, du méthyle ou de l'hydroxyle ; et
R¹² est de l'hydrogène ou du méthyle ; et
X est de l'azote ou le groupe C-H
OU 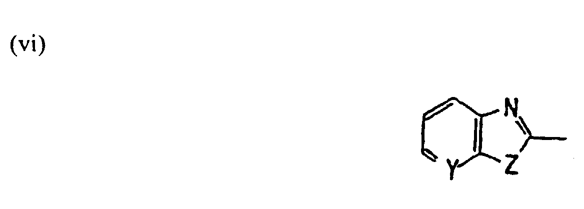 dans lequel
Z est de l'oxygène, du soufre ou le groupe N-H ; et
Y est le groupe C-H ou de l'azote
OU 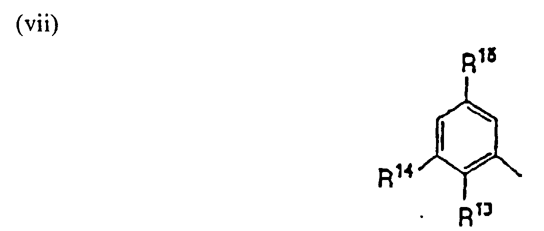 dans lequel
R¹³ est de l'hydrogène ou un groupe aminé ou carboxyle ; et
R¹⁴ est de l'hydrogène ou du méthyle ; et
R¹⁵ est de l'hydrogène ou du méthyle
OU 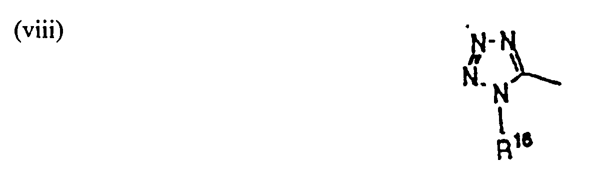 dans lequel R¹⁶ est un alcoyle ou un aryle
OU 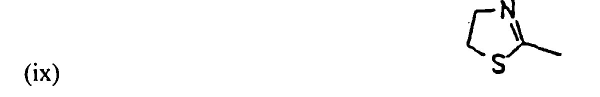 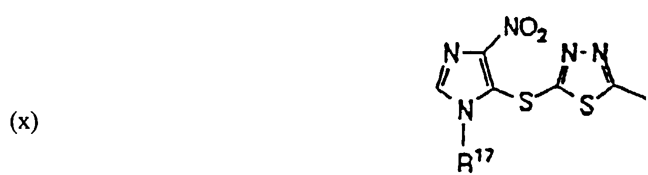 dans lequel R¹⁷ est un alcoyle ou un aryle
OU 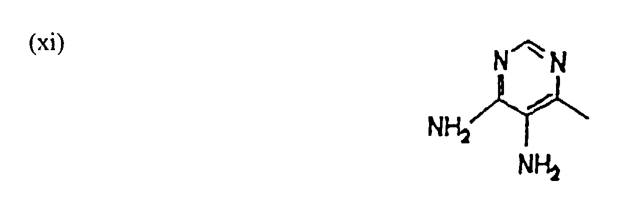 ou 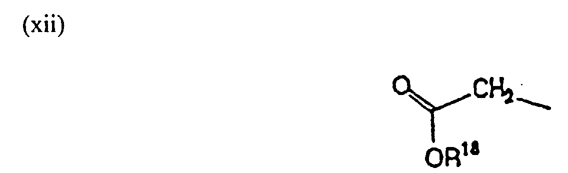 dans lequel R¹⁸ est un alcoyle ou un aryle
OU

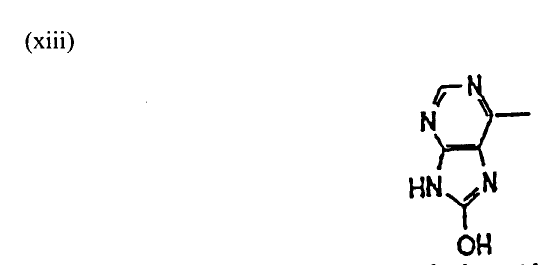

4. Un composé contenant la formule suivante : 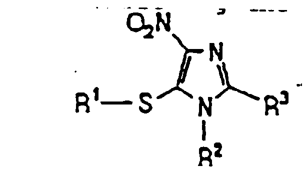 dans laquelle les groupes moléculaires R¹, R² et R³ sont définis comme l'un des ensembles suivants :
(i) R¹ est défini comme suit : 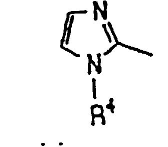 dans lequel
R⁴ est de l'hydrogène ou du méthyle ;
R² est de l'éthyle ; et
R³ est du méthyle
OU
R² est du méthyle et R³ de l'hydrogène ; et
R⁴ est du phényle ou du 1-naphtyle
OU
(ii) R¹ est défini comme suit : 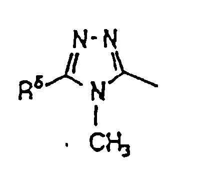 dans lequel
R², R³ et R⁵ forment l'un des combinaisons suivantes :
R² est du méthyle, R³ du 4-nitrophényle et R⁵ de l'hydrogène ; ou
R² est du méthyle, R³ de l'hydrogène et R⁵ du trifluorométhyle ; ou
R² est de l'éthyle, R³ du méthyle et R⁵ de l'hydrogène ; ou
R² est du méthyle, R³ de l'hydrogène et R⁵ du phényle ; ou
R² est du méthyle, R³ de l'hydrogène et R⁵ du 4-chlorophényle ; ou
R² est du méthyle, R³ de l'hydrogène et R⁵ du 4-méthoxyphényle ; ou
R² est du méthyle, R³ de l'hydrogène et R⁵ du 3-pyridyle
OU
(iii) R¹ est défini comme suit : 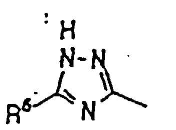 dans lequel
R² est du méthyle ; et
R³ est de l'hydrogène ; et
R⁶ est du phényle, du 4-chlorophényle ou du 4-méthoxyphényle
OU
(iv) R¹ est défini comme suit : 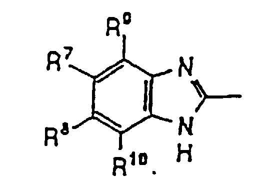 dans lequel
R² est du méthyle ; et
R³ est de l'hydrogène ; et
R⁷, R⁸, R⁹ et R¹⁰ forment l'une des combinaisons suivantes :
R⁹ et R¹⁰ sont tous deux de l'hydrogène et R⁷ et R⁸ sont tous deux des groupes méthoxy ; ou
R⁸, R⁹ et R¹⁰ sont tous de l'hydrogène et R⁷ du méthyle ; ou
R⁸, R⁹ et R¹⁰ sont tous de l'hydrogène et R⁷ un groupe nitré ; ou
R⁹ et R¹⁰ sont tous deux de l'hydrogène, R⁷ du fluor et R⁸ du chlore ; ou
R⁷ et R⁸ sont tous deux de l'hydrogène et R⁹ et R¹⁰ sont tous deux des groupes méthoxy
OU
(v) R¹ est défini comme suit : 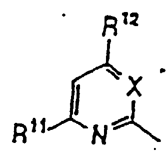 dans lequel
R² est du méthyle ; et
R³ est de l'hydrogène ; et
R¹¹, R¹² et X sont définis comme l'une des combinaisons suivantes
R¹¹ est un groupe hydroxy, R¹² de l'hydrogène et X de l'azote
OU
R² est de l'éthyle ; et
R³, R¹¹ et R¹² sont tous du méthyle ; et
X est de l'azote
OU
(vi) R¹ est défini comme suit : 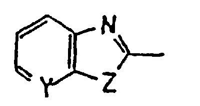 dans lequel
R² est du méthyle ; et
R³ est de l'hydrogène ; et
Y et Z sont définis comme l'une des combinaisons suivantes :
Y est le groupe C-H et Z de l'oxygène ; ou
Y est de l'azote et Z du soufre ; ou
Y est de l'azote et Z le groupe N-H
OU
(vii) R¹ est défini comme suit : 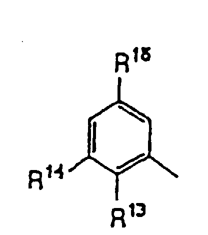 dans lequel
R² est du méthyle ; et
R³ est de l'hydrogène ; et
R¹³, R¹⁴ et R¹⁵ sont définis comme l'une des combinaisons suivantes :
R¹³ est de l'hydrogène et R¹⁴ et R¹⁵ sont tous deux du méthyle ;
OU
(viii) R¹ est défini comme suit : 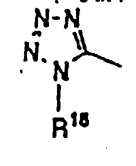 dans lequel
R² est du méthyle ; et
R³ est de l'hydrogène ; et
R16 est du méthyle ou du phényle
OU
(ix) R¹ est défini comme suit : 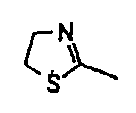 OU
(x) R¹ est défini comme suit : 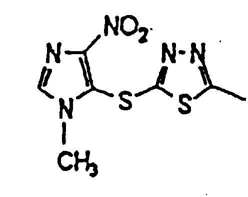 OU
(xi) R¹ est défini comme suit :

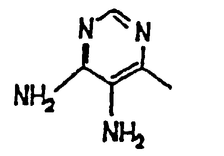

5. Une méthode pour produire un composé tel que revendiqué dans toutes les revendications précédentes au moyen d'une réaction du 5-chloro-4-notoimidazole ou ses dérivés avec un thiol, en présence d'un solvant et d'un carbonate.
